# EUROPEAN PATENT APPLICATION

(11) **EP 3 626 260 A1**
(43) Date of publication of application: **25.03.2020**
(21) Application number: 18801526.7
(22) Date of filing: 21.05.2018
(51) Int. Cl.: A61K 39/00, A61K 38/08, A61P 35/00, A61P 37/04, C07K 7/06

(54) **PEPTIDE VACCINE AND PEPTIDE VACCINE COMPOSITION FOR CRANIAL NERVE DISEASE**

(30) Priority: 19.05.2017 JP 2017100361
(71) Applicant: Keio University, Tokyo 108-8345 (JP)
(72) Inventor: TODA Masahiro, Tokyo 160-8582 (JP); TAMURA Ryota, Tokyo 160-8582 (JP)
(74) Representative: HGF Limited
(86) International application number: PCT/JP2018/019469
(87) International publication number: WO 2018/212358

(57) **Abstract**

A peptide vaccine for cranial nerve disease, containing a peptide that induces cytotoxic T-cells (CTLs) against Vascular Endothelial Growth Factor Receptor (VEGFR)-1-expressing cells, or a peptide that induces CTLs against VEGFR-2-expressing cells.

## Description

### Technical Field

The present invention relates to a peptide vaccine and a peptide vaccine composition for cranial nerve disease. Priority is claimed on Japanese Patent Application No. 2017-100361, filed May 19, 2017, the disclosure of which is incorporated herein by reference.

### Background Art

Cranial nerve diseases include neurofibromatosis type II (NF2), brain tumors, cerebrovascular diseases, autoimmune diseases, ophthalmic diseases and the like. For example, NF2 is an autosomal-dominant genetic disease that is caused by an abnormality in the gene for the tumor suppression factor merlin, located on the long arm of chromosome 22, at 22q12. A diagnosis for NF2 is established by using, as an indicator, the development of acoustic neuromas (more precisely, vestibular schwannomas) on both the left and right sides. In NF2, many tumors develop. Specifically, schwannomas develop in cranial nerves, spinal nerves and peripheral nerves, and further thereto, meningiomas, ependymomas, gliomas and the like develop in the cranium and in the spine. NF2 often occurs at an age in the tens or the twenties, progresses quickly, causes hearing loss, hemiplegia or the like, and when the growth of intracranial tumors cannot be controlled, has a high risk of mortality.

Currently, NF2 is treated by surgical tumorectomy and stereotactic radiation therapy. However, the schwannomas in NF2 patients are hemorrhagic tumors, so there is a high probability that surgery will damage the surrounding nerves. In particular, when there are multiple tumors, it is not possible to take an active approach to surgery. Additionally, although radiation therapy is effective against small tumors, it is often difficult to achieve long-term control, and there is also the risk of malignant transformation.

It is known that vascular endothelial growth factor receptor (VEGFR) is a type of receptor-type tyrosine kinase that is involved in the action of VEGF, which is a ligand thereof.

VEGFR includes the three types, VEGFR-1, VEGFR-2 and VEGFR-3, in addition to which sVEGFR-1, sVEGFR-2 and sVEGFR-3 are known as soluble VEGFRs. Of these, it is known that VEGFR-1 and VEGFR-2 are expressed in vascular endothelial cells, and play a central role in angiogenesis.

Conventionally, peptides that induce cytotoxic T-cells (CTLs) against VEGFR-1-expressing cells and peptides that induce CTLs against VEGFR-2-expressing cells have been developed (see, for example, Patent Documents 1 and 2).

### Citation List

### Patent Literature

Patent Document 1] JP 4931154 B
Patent Document 2] JP 3971769 B

### Summary of Invention

### Technical Problem

Research is being performed towards applying the partial peptides of VEGFR described in Patent Document 1, 2, etc., as peptide vaccines, to cancer therapy. However, for example, the efficacy of VEGFR-2 partial peptides was not confirmed in phase III clinical trials on recurrent pancreatic cancer. Cancer has a fast growth rate, and in most cases, immunity is suppressed by the administration of anti-cancer agents, so it is not easy to apply peptide vaccines to cancer therapy.

Under these circumstances, an objective of the present invention is to provide a peptide vaccine and a peptide vaccine composition that are able to effectively treat cranial nerve disease.

### Solution to Problem

The present invention includes the embodiments indicated below.
[1] A peptide vaccine for cranial nerve disease, comprising a peptide that induces cytotoxic T-cells (CTLs) against VEGFR-1-expressing cells, or a peptide that induces CTLs against VEGFR-2-expressing cells.
[2] The peptide vaccine as in [1], wherein the peptide that induces CTLs against VEGFR-1-expressing cells is an HLA-A^{∗}24:02-binding peptide.
[3] The peptide vaccine as in [2], wherein the peptide that induces CTLs against VEGFR-1-expressing cells has the amino acid sequence as set forth in SEQ ID NO:1, or an amino acid sequence obtained by deletion, substitution or addition of one or a plurality of amino acids with respect to the amino acid sequence as set forth in SEQ ID NO:1.
[4] The peptide vaccine as in [1], wherein the peptide that induces CTLs against VEGFR-1-expressing cells is an HLA-A^{∗}02:01-binding peptide.
[5] The peptide vaccine as in [4], wherein the peptide that induces CTLs against VEGFR-1-expressing cells has the amino acid sequence as set forth in any one of SEQ ID NO:2 to 4, or an amino acid sequence obtained by deletion, substitution or addition of one or a plurality of amino acids with respect to the amino acid sequence as set forth in any one of SEQ ID NO:2 to 4.
[6] The peptide vaccine as in [1], wherein the peptide that induces CTLs against VEGFR-1-expressing cells is an HLA-A^{∗}02:06-binding peptide or an HLA-A^{∗}02:07-binding peptide.
[7] The peptide vaccine as in [1], wherein the peptide that induces CTLs against VEGFR-2-expressing cells is an HLA-A^{∗}24:02-binding peptide.
[8] The peptide vaccine as in [7], wherein the peptide that induces CTLs against VEGFR-2-expressing cells has the amino acid sequence as set forth in any one of SEQ ID NO:5 to 10, or an amino acid sequence obtained by deletion, substitution or addition of one or a plurality of amino acids with respect to the amino acid sequence as set forth in any one of SEQ ID NO:5 to 10.
[9] The peptide vaccine as in [1], wherein the peptide that induces CTLs against VEGFR-2-expressing cells is an HLA-A^{∗}02:01-binding peptide.
[10] The peptide vaccine as in [9], wherein the peptide that induces CTLs against VEGFR-2-expressing cells has the amino acid sequence as set forth in any one of SEQ ID NO:11 to 16, or an amino acid sequence obtained by deletion, substitution or addition of one or a plurality of amino acids with respect to the amino acid sequence as set forth in any one of SEQ ID NO:11 to 16.
[11] The peptide vaccine as in [1], wherein the peptide that induces CTLs against VEGFR-2-expressing cells is an HLA-A^{∗}02:06-binding peptide or an HLA-A^{∗}02:07-binding peptide.
[12] The peptide vaccine as in any one of [1] to [11], wherein the cranial nerve disease is neurofibromatosis type II.
[13] A peptide vaccine composition for tumors in NF2 patients, containing the peptide vaccine as in any one of claims [1] to [12], and a pharmaceutically acceptable carrier.

### Advantageous Effects of Invention

According to the present invention, it is possible to provide a peptide vaccine and a peptide vaccine composition that are able to effectively treat cranial nerve disease.

### Brief Description of Drawings

Fig. 1(a) to (e) show photographs indicating the results of imaging of a meningioma in a patient in Case 1 by means of contrast-enhanced head MRI examination in Experimental Example 2.
Fig. 2 is a graph showing the results of measurement of the volume of the meningioma on each examination day, based on the results of the contrast-enhanced head MRI examination in Experimental Example 2.
Fig. 3(a) to (e) show photographs indicating the results of imaging of right and left vestibular schwannomas in the patient in Case 1 by means of contrast-enhanced head MRI examination in Experimental Example 2.
Fig. 4(a) is a graph showing the results of measurement of the volume of the right vestibular schwannoma on each examination day, based on the results of the contrast-enhanced head MRI examination in Experimental Example 2. Fig. 4(b) is a graph showing the results of measurement of the volume of the left vestibular schwannoma on each examination day, based on the results of the contrast-enhanced head MRI examination in Experimental Example 2.
Fig. 5(a) to (f) show photographs indicating the results of imaging of a trigeminal scwhannoma in a patient in Case 2 by means of contrast-enhanced head MRI examination in Experimental Example 3.
Fig. 6 is a graph showing the results of measurement of the volume of the trigeminal schwannoma on each examination day, based on the results of the contrast-enhanced head MRI examination in Experimental Example 3.
Fig. 7(a) to (f) show photographs indicating the results of imaging of the right and left vestibular schwannomas in the patient in Case 2 by means of contrast-enhanced head MRI examination in Experimental Example 3.
Fig. 8(a) is a graph showing the results of measurement of the volume of the right vestibular schwannoma on each examination day, based on the results of the contrast-enhanced head MRI examination in Experimental Example 3. Fig. 8(b) is a graph showing the results of measurement of the volume of the left vestibular schwannoma on each examination day, based on the results of the contrast-enhanced head MRI examination in Experimental Example 3.
Fig. 9 is a graph showing the results of measurement of the tumor blood volume in the meningioma in Experimental Example 2.
Fig. 10(a) is a graph showing the results of measurement of the tumor blood volume in the right vestibular schwannoma in Experimental Example 2. Fig. 10(b) is a graph showing the results of measurement of the tumor blood volume in the left vestibular schwannoma in Experimental Example 2.
Fig. 11 is a graph showing the results of measurement of the tumor blood volume in the trigeminal schwannoma in Experimental Example 3.
Fig. 12(a) is a graph showing the results of measurement of the tumor blood volume in the right vestibular schwannoma in Experimental Example 3. Fig. 12(b) is a graph showing the results of measurement of the tumor blood volume in the left vestibular schwannoma in Experimental Example 3.
Fig. 13(a) and (b) show graphs indicating the results of speech intelligibility tests performed on the patient in Case 2 in Experimental Example 3.
Fig. 14(a) to (f) show photographs indicating the results of imaging of a left vestibular schwannoma and a meningioma in a patient in Case 5 by means of contrast-enhanced head MRI examination in Experimental Example 4.
Fig. 15(a) is a graph showing the results of measurement of the volume of the left vestibular schwannoma on each examination day, based on the results of the contrast-enhanced head MRI examination in Experimental Example 4. Fig. 15(b) is a graph showing the results of measurement of the volume of the meningioma on each examination day, based on the results of the contrast-enhanced head MRI examination in Experimental Example 4.
Fig. 16(a) is a graph showing the results of measurement of the tumor blood volume in the right vestibular schwannoma in Experimental Example 4. Fig. 16(b) is a graph showing the results of measurement of the tumor blood volume in the left vestibular schwannoma in Experimental Example 4.
Fig. 17(a) to (d) show photographs indicating the results of imaging of bilateral vestibular schwannomas in a patient in Case 6 by means of contrast-enhanced head MRI examination in Experimental Example 5.
Fig. 18(a) is a graph showing the results of measurement of the volume of the right vestibular schwannoma on each examination day, based on the results of the contrast-enhanced head MRI examination in Experimental Example 5. Fig. 18(b) is a graph showing the results of measurement of the volume of the left vestibular schwannoma on each examination day, based on the results of the contrast-enhanced head MRI examination in Experimental Example 5.
Fig. 19(a) is a graph showing the results of measurement of the tumor blood volume in the right vestibular schwannoma in Experimental Example 6. Fig. 19(b) is a graph showing the results of measurement of the tumor blood volume in the left vestibular schwannoma in Experimental Example 6.
Fig. 20(a) to (d) show photographs indicating the results of imaging of a meningioma in the patient in Case 6 by means of contrast-enhanced head MRI examination in Experimental Example 6.
Fig. 21 is a graph showing the results of measurement of the volume of the meningioma on each examination day based on the results of contrast-enhanced head MRI examination in Experimental Example 6.
Fig. 22 is a graph showing the results of measurement of the tumor blood volume in the meningioma in Experimental Example 6.
Fig. 23 shows a graph indicating the results of quantitative RT-PCR in Experimental Example 7.
Fig. 24(a) shows a microscope photograph indicating the results of immunostaining obtained before administration of the peptide vaccine composition in Experimental Example 7. Fig. 24(b) shows a microscope photograph indicating the results of immunostaining obtained after administration of the peptide vaccine composition in Experimental Example 7.
Fig. 25(a) shows a microscope photograph indicating the results of immunostaining obtained before administration of the peptide vaccine composition in Experimental Example 7. Fig. 25(b) shows a microscope photograph indicating the results of immunostaining obtained after administration of the peptide vaccine composition in Experimental Example 7.
Fig. 26 shows a fluorescence microscope photograph indicating the results of immunostaining in Experimental Example 7.

### Description of Embodiments

### [Peptide Vaccine]

In one embodiment, the present invention provides a peptide vaccine for cranial nerve disease, consisting of a peptide that induces CTLs against VEGFR-1-expressing cells, or a peptide that induces CTLs against VEGFR-2-expressing cells.

In the present description, examples of cranial nerve diseases include cranial nerve diseases that are associated with angiogenesis, more specifically, tumors that develop in neurofibromatosis type II (NF2) patients, brain tumors, cerebrovascular diseases, autoimmune diseases, ophthalmic diseases and the like.

Examples of tumors that develop in NF2 patients include schwannomas, meningiomas, ependymomas, gliomas and the like. Schwannomas include schwannomas that develop in cranial nerves, spinal nerves and peripheral nerves, more specifically, vestibular schwannomas, trigeminal schwannomas and the like. Additionally, examples of meningiomas, ependymomas and gliomas include tumors forming in the cranium and tumors forming in the spine.

Additionally, examples of brain tumors include gliomas, meningiomas, schwannomas, pituitary adenomas, craniopharyngiomas, chordomas, ependymomas, hemangioblastomas, hemangiopericytomas, metastatic brain tumors, medulloblastomas, malignant lymphomas and the like. Additionally, examples of cerebrovascular diseases include cerebrovascular malformation, hereditary telangiectasia and the like. Additionally, examples of autoimmune diseases include neuromyelitis optica, giant-cell arteritis and the like. Additionally, examples of ophthalmic diseases include age-related macular degeneration, diabetic retinopathy and the like.

For example, when a schwannoma or a meningioma forms in the cranium, the patient's life is at risk. However, in the past, there was no effective method for treating such a tumor.

In contrast therewith, as explained below in the examples, the inventors demonstrated that it is possible to effectively treat cranial nerve disease by administering the peptide vaccine of the present embodiment to a cranial nerve disease patient.

Human VEGFR-1 is a cell membrane protein consisting of 1338 amino acids. Human VEGFR-1 is a vascular endothelial growth factor receptor that is indispensable for neovascular growth and is highly expressed in tumor neovascular endothelial cells in solid tumors. Additionally, human VEGFR-2 is a cell membrane protein consisting of 1356 amino acids. Human VEGFR-2 is highly expressed in tumor neovascular endothelial cells in solid tumors, and is involved in the growth and migration of vascular endothelial cells.

The peptide vaccine of the present embodiment is able to induce CTLs against VEGFR-1-expressing cells or VEGFR-2-expressing cells. It is thought that the CTLs that are induced in the patient's body by administering the peptide vaccine of the present embodiment specifically destroy the blood vessels in tumors that have developed in cranial nerve diseases, thereby resulting in tumor growth suppression. Furthermore, it is thought that some of the CTLs continue to exist as memory T cells, thereby also providing long-term therapeutic effects even after the administration of the peptide vaccine has been completed.

In the present description, effectively treating a tumor may, for example, refer to suppressing increases in tumor volume, or to reducing tumor volume.

It is possible to use a known peptide as the peptide for inducing CTLs against VEGFR-1-expressing cells or the peptide for inducing CTLs against VEGFR-2-expressing cells. Known peptides include those that have been confirmed to be safe when administered to humans in clinical tests. Such peptides are easy to apply clinically because it is possible to skip some of the clinical tests.

In the peptide vaccine of the present embodiment, the length of the peptide may be less than approximately 40 amino acids, may be less than approximately 20 amino acids, and may be less than approximately 15 amino acids. For example, the length of the peptide may be 8 to 11 amino acids, may be 9 amino acids, or may be 10 amino acids.

In the peptide vaccine of the present embodiment, the peptide may be prepared by means of a known method. For example, the peptide may be prepared by recombinant DNA technology, or may be prepared by chemical synthesis.

Additionally, the peptide may be modified in various ways as long as it has the activity of inducing CTLs. Specifically, for example, a sugar chain, a fatty acid, a polyalkylene glycol chain or the like may be bonded to the peptide. Alternatively, the peptide may include a D-amino acid. Alternatively, a side chain of the peptide may be modified by oxidation, phosphorylation or the like. Alternatively, the peptide may form a trifluoroacetic acid salt, an acetic acid salt, a hydrochloric acid salt or the like.

Human leukocyte antigens (HLA) are membrane proteins that are also called histocompatibility antigens, and they play an important role in the immune mechanism. It is known that there are many types of HLAs. In order for the peptide vaccine to function effectively, the peptide must have binding affinity to an HLA that is of the same type as the patient's HLA type.

Humans having HLA types such as HLA-A^{∗}24:02, HLA-A^{∗}02:01, HLA-A^{∗}02:06 and HLA-A^{∗}02:07 are relatively common, particularly among the Japanese. The peptide vaccine of the present embodiment is HLA-A^{∗}24:02-binding (restricting) or HLA-A^{∗}02:01-binding, and is also expected to bind to HLA-A^{∗}02:06 and HLA-A^{∗}02:07. Thus, the peptide vaccine can function effectively in many Japanese patients.

Therefore, in the peptide vaccine of the present embodiment, the peptide that induces CTLs against VEGFR-1-expressing cells may be HLA-A^{∗}24:02-binding, may be HLA-A^{∗}02:01-binding, may be HLA-A^{∗}02:06-binding, or may be HLA-A^{∗}02:07-binding. Additionally, the peptide that induces CTLs against VEGFR-2-expressing cells may be HLA-A^{∗}24:02-binding, may be HLA-A^{∗}02:01-binding, may be HLA-A^{∗}02:06-binding, or may be HLA-A^{∗}02:07-binding. Hereinafter, more specific peptides that can be used in the peptide vaccine of the present embodiment will be explained.

### (HLA-A^{∗}24:02-binding peptides that induce CTLs against VEGFR-1-expressing cells)

HLA-A^{∗}24:02-binding peptides that can induce CTLs against VEGFR-1-expressing cells are already known. Such peptides include, for example, the peptide consisting of the amino acid sequence as set forth in SEQ ID NO:1.

In this case, the peptide consisting of the amino acid sequence as set forth in SEQ ID NO:1 may include alterations as long as it is able to induce CTLs against VEGFR-1-expressing cells. The peptide that induces CTLs against VEGFR-1-expressing cells may, for example, be a peptide consisting of an amino acid sequence obtained by the deletion, substitution or addition of one or a plurality of amino acids with respect to the amino acid sequence as set forth in SEQ ID NO:1.

In this case, one or a plurality is preferably one, two or three. Examples of amino acid substitutions include the substitution of the second amino acid from the N-terminus by phenylalanine, tyrosine, methionine or tryptophan, and the substitution of the amino acid at the C-terminus by phenylalanine, leucine, isoleucine, tryptophan or methionine. Additionally, examples of amino acid addition include the addition of one or two amino acids to the N-terminus or the C-terminus.

### (HLA-A^{∗}02:01-binding peptides that induce CTLs against VEGFR-1-expressing cells)

HLA-A^{∗}02:01-binding peptides that can induce CTLs against VEGFR-1-expressing cells are already known. Such peptides include, for example, the peptides consisting of the amino acid sequences as set forth in SEQ ID NO:2 to 4.

In this case, a peptide consisting of the amino acid sequence as set forth in any one of SEQ ID NO:2 to 4 may include alterations as long as it is able to induce CTLs against VEGFR-1-expressing cells. The peptide that induces CTLs against VEGFR-1-expressing cells may, for example, be a peptide consisting of an amino acid sequence obtained by the deletion, substitution or addition of one or a plurality of amino acids with respect to the amino acid sequence as set forth in any one of SEQ ID NO:2 to 4.

In this case, one or a plurality is preferably one, two or three. Examples of amino acid substitutions include the substitution of the second amino acid from the N-terminus by leucine or methionine, and the substitution of the amino acid at the C-terminus by valine or leucine. Additionally, examples of amino acid addition include the addition of one or two amino acids to the N-terminus or the C-terminus.

### (HLA-A^{∗}24:02-binding peptides that induce CTLs against VEGFR-2-expressing cells)

HLA-A^{∗}24:02-binding peptides that can induce CTLs against VEGFR-2-expressing cells are already known. Such peptides include, for example, the peptides consisting of the amino acid sequences as set forth in SEQ ID NO:5 to 10.

In this case, a peptide consisting of the amino acid sequence as set forth in any one of SEQ ID NO:5 to 10 may include alterations as long as it is able to induce CTLs against VEGFR-2-expressing cells. The peptide that induces CTLs against VEGFR-2-expressing cells may, for example, be a peptide consisting of an amino acid sequence obtained by the deletion, substitution or addition of one or a plurality of amino acids with respect to the amino acid sequence as set forth in any one of SEQ ID NO:5 to 10.

In this case, one or a plurality is preferably one, two or three. Examples of amino acid substitutions include the substitution of the second amino acid from the N-terminus by phenylalanine, tyrosine, methionine or tryptophan, and the substitution of the amino acid at the C-terminus by phenylalanine, leucine, isoleucine, tryptophan or methionine. Additionally, examples of amino acid addition include the addition of one or two amino acids to the N-terminus or the C-terminus.

### (HLA-A^{∗}02:01-binding peptides that induce CTLs against VEGFR-2-expressing cells)

HLA-A^{∗}02:01-binding peptides that can induce CTLs against VEGFR-2-expressing cells are already known. Such peptides include, for example, the peptides consisting of the amino acid sequences as set forth in SEQ ID NO:11 to 16.

In this case, a peptide consisting of the amino acid sequence as set forth in any one of SEQ ID NO:11 to 16 may include alterations as long as it is able to induce CTLs against VEGFR-2-expressing cells. The peptide that induces CTLs against VEGFR-2-expressing cells may, for example, be a peptide consisting of an amino acid sequence obtained by the deletion, substitution or addition of one or a plurality of amino acids with respect to the amino acid sequence as set forth in any one of SEQ ID NO:11 to 16.

In this case, one or a plurality is preferably one, two or three. Examples of amino acid substitutions include the substitution of the second amino acid from the N-terminus by leucine or methionine, and the substitution of the amino acid at the C-terminus by valine or leucine. Additionally, examples of amino acid addition include the addition of one or two amino acids to the N-terminus or the C-terminus.

For example, a peptide consisting of the amino acid sequence (SEQ ID NO:16) obtained by substituting a leucine for the second amino acid from the N-terminus in the amino acid sequence as set forth in SEQ ID NO: 12 is a suitable example. For example, it has been confirmed that CTLs that are induced by stimulation by the peptide consisting of the amino acid sequence as set forth in SEQ ID NO:16 are able recognize and damage the pre-alteration peptides (SEQ ID NO:12).

### [Peptide Vaccine Composition]

In one embodiment, the present invention provides a peptide vaccine composition for cranial nerve disease, containing the above-mentioned peptide vaccine, and a pharmaceutically acceptable carrier.

The above-mentioned peptide vaccine may be directly administered to a patient, or may be administered to a patient in the form of the peptide vaccine composition of the present embodiment. As explained below in the examples, the inventors demonstrated that it is possible to effectively treat cranial nerve disease by administering the peptide vaccine of the present embodiment to a cranial nerve disease patient.

The peptide vaccine composition of the present embodiment may contain just one or both of the peptide that induces CTLs against VEGFR-1-expressing cells and the peptide that induces CTLs against VEGFR2-expressing cells.

The peptide vaccine composition of the present embodiment may be formulated by means of a generally known pharmaceutical production method, by being mixed with a pharmaceutically acceptable carrier. For example, it may be formulated in the form of tablets, capsules, elixirs, microcapsules or the like and administered orally, or may be formulated in the form of an injection, a suppository, a topical skin preparation or the like and administered non-orally. Examples of topical skin preparations include ointments, patches or the like.

Examples of pharmaceutically acceptable carriers include adjuvants, solvents for injection, excipients, agglutinants and the like. As the adjuvant, it is possible to use, without any particular restrictions, any substance that is generally used as an adjuvant for vaccine formulations. Examples include, but are not limited to, precipitated adjuvants such as sodium hydroxide, aluminum hydroxide, calcium phosphate, aluminum phosphate, alum, pepesu and carboxyvinyl polymer, oil-based adjuvants such as liquid paraffin, lanolin, incomplete Freund's adjuvant and complete Freund's adjuvant, and the like.

As the injectable solvent, it is possible to use, without any particular restrictions, any substance that is generally used in injections. Examples include, but are not limited to, isotonic solutions including auxiliary agents such as physiological saline solution, glucose, D-sorbitol, D-mannose, D-mannitol, sodium chloride and the like. The injectable solvent may contain an alcohol such as ethanol; a polyalcohol such as propylene glycol and polyethylene glycol; a non-ionic surfactant such as polysorbate 80 (trademark), HCO-50 or the like.

As the excipient, it is possible to use, without any particular restrictions, any substance that is generally used in pharmaceuticals. Examples include, but are not limited to, starches, crystalline cellulose and the like.

As the agglutinant, it is possible to use, without any particular restrictions, any substance that is generally used in patches or the like. Examples include, but are not limited to, rubber-based agglutinants, silicone-based agglutinants and the like.

The peptide vaccine composition of the present embodiment may be administered together with an active ingredient, such as another anti-cancer agent, or may contain an active ingredient, such as another anti-cancer agent.

The above-mentioned peptide vaccine or the peptide vaccine composition of the present embodiment may be administered to a patient, for example, by oral delivery, intradermal delivery, subcutaneous delivery, intravenous injection or the like, and may be administered systemically, or administered locally in the vicinity of a target tumor.

Additionally, the dosage may be appropriately adjusted in accordance with the age and weight of the patient, the symptoms, the administration method or the like. In a normal adult (body weight 60 kg), each dose may deliver, for example, 0.001 mg to 1000 mg, for example, 0.001 mg to 1000 mg, and for example, 0.1 mg to 10 mg of the active ingredient (peptide).

The number of doses of the peptide vaccine or peptide vaccine composition that are administered to a patient may be a single dose, but multiple doses are preferred in order to efficiently induce CTLs. The administration interval in the case of multiple doses may, for example, be a few days to a few months.

### [Other Embodiments]

In one embodiment, the present invention provides a method for treating cranial nerve disease that includes administering, to a patient requiring treatment, an effective amount of a peptide that induces CTLs against VEGFR-1-expressing cells or a peptide that induces CTLs against VEGFR-2-expressing cells.

In one embodiment, the present invention provides a peptide that induces CTLs against VEGFR-1-expressing cells or a peptide that induces CTLs against VEGFR-2-expressing cells for treating cranial nerve disease.

In one embodiment, the present invention provides the use of a peptide that induces CTLs against VEGFR-1-expressing cells or a peptide that induces CTLs against VEGFR-2-expressing cells for producing a therapeutic agent for cranial nerve disease.

Examples of the peptide that induces CTLs against VEGFR-1-expressing cells and the peptide that induces CTLs against VEGFR-2-expressing cells in these embodiments include those mentioned above. Additionally, the abovementioned peptides may be in the form of a composition containing a pharmaceutically acceptable carrier. Examples of pharmaceutically acceptable carriers include those mentioned above.

### Examples

Next, the present invention will be explained in further detail by indicating experimental examples, but the present invention is not limited to the experimental examples below.

### [Experimental Example 1]

### (Preparation of peptide vaccine composition)

By mixing and emulsifying 2 mg of a peptide consisting of the amino acid sequence as set forth in SEQ ID NO:1, 2 mg of a peptide consisting of the amino acid sequence as set forth in SEQ ID NO:5 and 1 mL of incomplete Freund's adjuvant ("Montanide ISA51", SEPPIC), 2 mL of a peptide vaccine composition was prepared.

### [Experimental Example 2]

### (Administration of peptide vaccine composition to patient in Case 1)

The patient in Case 1 was a 30-year-old male. The HLA type of the patient in Case 1 was HLA-A^{∗}24:02. This patient had bilateral vestibular schwannomas, a trigeminal schwannoma and a meningioma in the cranium, and also had numerous schwannomas in the spine and on the skin. He had already completely lost hearing in both ears and was in a bedridden state. He had undergone four tumorectomy operations and three gamma-knife radiosurgery operations, including in the cranium and in the spine, but it was difficult to control the increase in the tumors and the tumors were increasing year by year. The patient in Case 1 was administered a peptide vaccine composition prepared in the same manner as that in Experimental Example 1.

### (Dosing schedule)

Each dose involved subcutaneous delivery of 1 mL of the peptide vaccine composition in the periphery of the bilateral axillary and inguinal lymph nodes of the patient. As the initial administration, four doses were administered, once weekly, for a period of one month after administration began. Thereafter, as boost administrations, four doses were administered, once a month. Due to the above dosing schedule, the peptide vaccine composition was administered a total of eight times in a five-month period from the beginning of administration.

### (Image analysis)

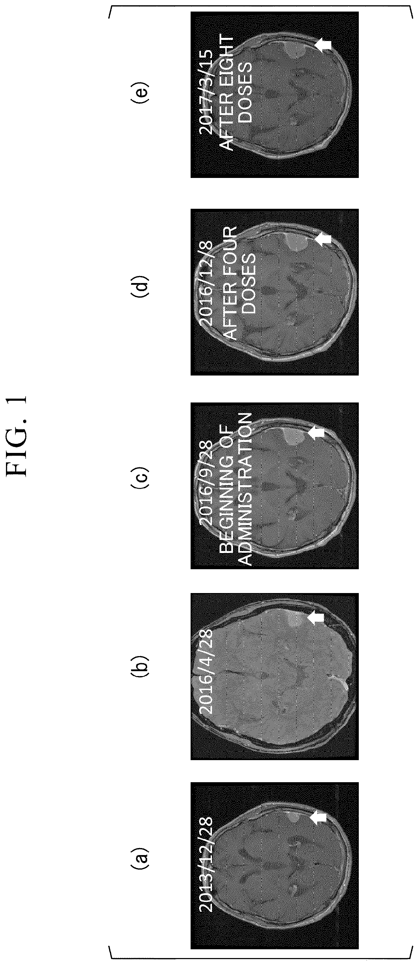

The effects of the administration of the peptide vaccine composition on the patient's tumors were investigated by means of contrast-enhanced head MRI examination. Fig. 1(a) to (e) show photographs indicating the results of imaging of the meningioma in the patient in Case 1 by means of contrast-enhanced head MRI examination. The dates on which the images were taken are indicated on the upper part of each photograph. Fig. 1(a) and (b) show the results before administration of the peptide vaccine composition, Fig. 1(c) shows the results at the time the administration of the peptide vaccine composition began, Fig. 1(d) shows the results after four doses of the peptide vaccine composition were administered, and Fig. 1(e) shows the results after eight doses of the peptide vaccine composition were administered. In Fig. 1(a) to (e), the arrows indicate the meningioma.

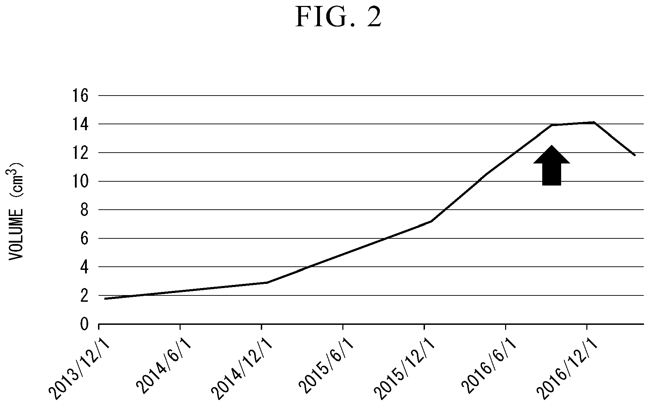

Additionally, Fig. 2 is a graph showing the results of measurement of the volume of the meningioma on each examination day, based on the results of the contrast-enhanced head MRI examination. In Fig. 2, the arrow indicates the time at which the administration of the vaccine composition began.

As a result thereof, it was demonstrated that, after eight doses of the peptide vaccine composition were administered, the growth of many tumors in the cranium, which had been on an increasing trend until that point, was stopped. Additionally, it was demonstrated that the volume of the meningioma after eight doses of the peptide vaccine composition were administered was reduced by approximately 10% in comparison to the volume at the time the administration of the peptide vaccine composition began.

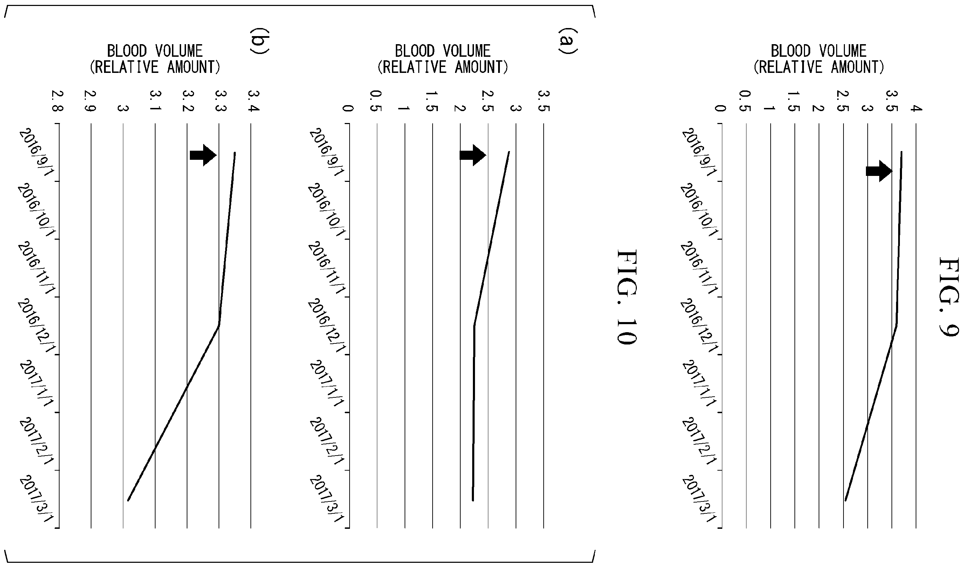

Additionally, Fig. 9 is a graph showing the results of measurement of the tumor blood volume in the meningioma on each examination day based on the results of contrast-enhanced head CT examination. In Fig. 9, the arrow indicates the time at which the administration of the vaccine composition began. As a result thereof, it was demonstrated that the tumor blood volume decreased after the peptide vaccine composition was administered.

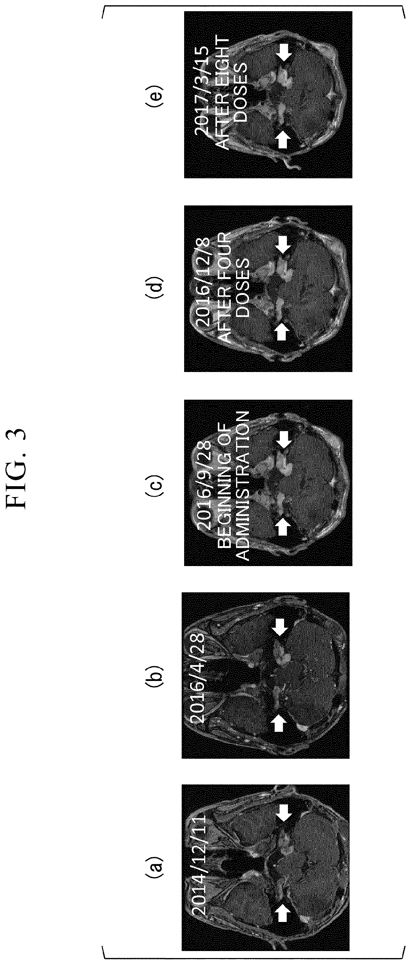

Additionally, Fig. 3(a) to (e) show photographs indicating the results of imaging of the right and left vestibular schwannomas in the patient in Case 1 by means of contrast-enhanced head MRI examination. The dates on which the images were taken are indicated on the upper part of each photograph. Fig. 3(a) and (b) show the results before administration of the peptide vaccine composition, Fig. 3(c) shows the results at the time the administration of the peptide vaccine composition began, Fig. 3(d) shows the results after four doses of the peptide vaccine composition were administered, and Fig. 3(e) shows the results after eight doses of the peptide vaccine composition were administered. In Fig. 3(a) to (e), the arrows indicate vestibular schwannomas.

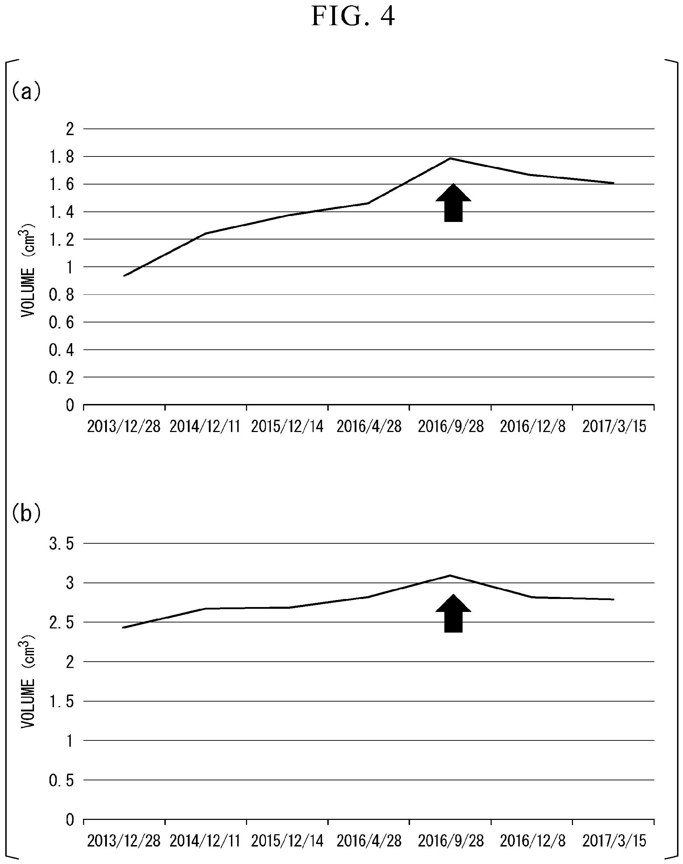

Additionally, Fig. 4(a) is a graph showing the results of measurement of the volume of the right vestibular schwannoma on each examination day, based on the results of the contrast-enhanced head MRI examination, and Fig. 4(b) is a graph showing the results of measurement of the volume of the left vestibular schwannoma on each examination day, based on the results of the contrast-enhanced head MRI examination. In Fig. 4(a) and (b), the arrows indicate the time at which the administration of the vaccine composition began.

As a result thereof, it was demonstrated that, after the administration of the peptide vaccine composition began, the growth of both the right and left vestibular schwannomas, which had been on an increasing trend until that point, was stopped. Additionally, it was demonstrated that the volumes of the right and left vestibular schwannomas after eight doses of the peptide vaccine composition were administered were reduced by approximately 10% in comparison to the volumes at the time the administration of the peptide vaccine composition began.

Additionally, Fig. 10(a) is a graph showing the results of measurement of the tumor blood volume in the right vestibular schwannoma on each examination day based on the results of contrast-enhanced head CT examination, and Fig. 10(b) is a graph showing the results of measurement of the tumor blood volume in the left vestibular schwannoma on each examination day based on the results of contrast-enhanced head CT examination. In Fig. 10(a) and (b), the arrows indicate the time at which the administration of the vaccine composition began. As a result thereof, it was demonstrated that the tumor blood volumes decreased after the peptide vaccine composition was administered.

### [Experimental Example 3]

### (Administration of peptide vaccine composition to patient in Case 2)

The patient in Case 2 was a 22-year-old female. The HLA type of the patient in Case 2 was HLA-A^{∗}24:02. This patient was diagnosed with NF2 after developing hearing problems. She had bilateral vestibular schwannomas and a trigeminal schwannoma, and also had numerous schwannomas in the spine and on the skin. She had already lost hearing in the right ear and, though still able to hold conversations by using a hearing aid in the left ear, her hearing was gradually declining. She had undergone three tumorectomy operations and one gamma-knife radiosurgery operation, but it was difficult to control the increase in the tumors and the tumors were gradually increasing.

The patient in Case 2 was administered a peptide vaccine composition prepared in the same manner as that in Experimental Example 1. The dosing schedule and the image analysis were the same as those in Experimental Example 2.

### (Image analysis)

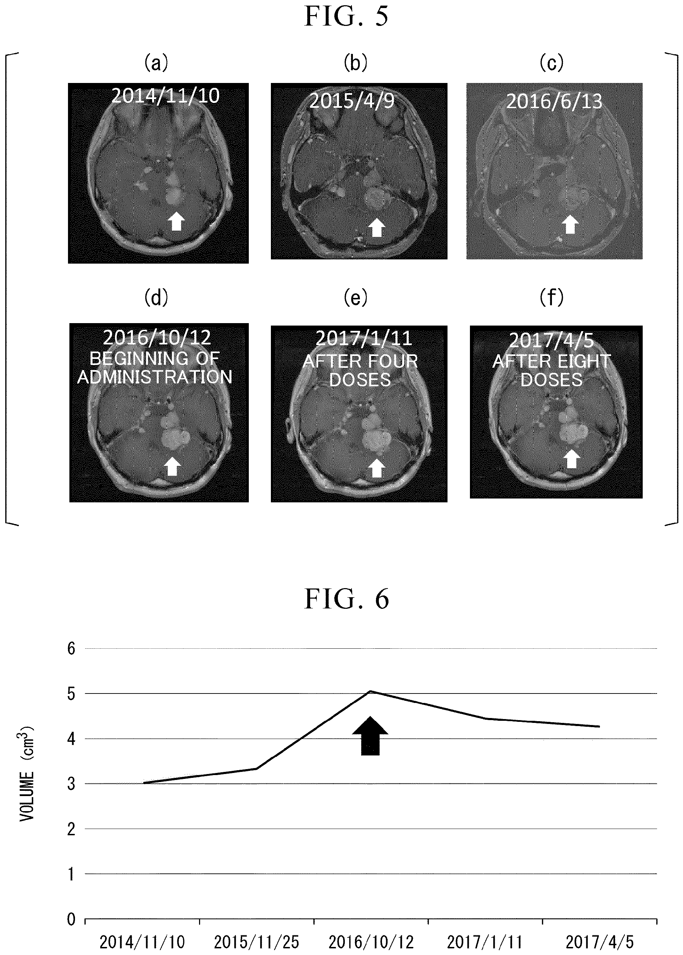

The effects of the administration of the peptide vaccine composition on the patient's tumors were investigated by means of contrast-enhanced head MRI examination. Fig. 5(a) to (f) show photographs indicating the results of imaging of the trigeminal scwhannoma in the NF2 patient in Case 2 by means of contrast-enhanced head MRI examination. The dates on which the images were taken are indicated on the upper part of each photograph. Fig. 5(a) to (c) show the results before administration of the peptide vaccine composition, Fig. 5(d) shows the results at the time the administration of the peptide vaccine composition began, Fig. 5(e) shows the results after four doses of the peptide vaccine composition were administered, and Fig. 5(f) shows the results after eight doses of the peptide vaccine composition were administered. In Fig. 5(a) to (f), the arrows indicate the trigeminal schwannoma.

Additionally, Fig. 6 is a graph showing the results of measurement of the volume of the trigeminal schwannoma on each examination day, based on the results of the contrast-enhanced head MRI examination. In Fig. 6, the arrow indicates the time at which the administration of the vaccine composition began.

As a result thereof, it was demonstrated that, after the peptide vaccine composition was administered, the growth of the trigeminal schwannoma, which had been on an increasing trend until that point, was stopped. Additionally, it was demonstrated that the volume of the trigeminal schwannoma after eight doses of the peptide vaccine composition were administered was reduced by approximately 14% in comparison to the volume at the time the administration of the peptide vaccine composition began.

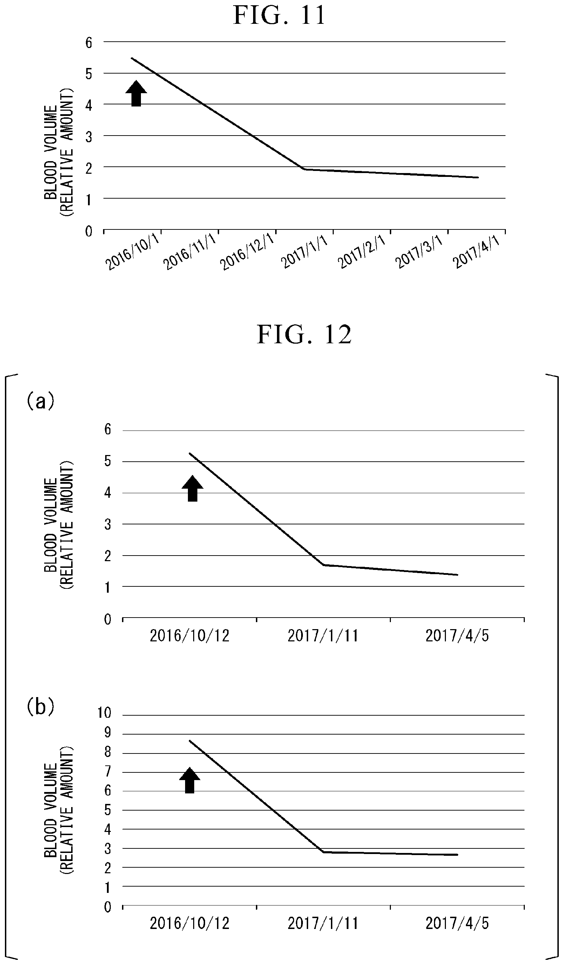

Additionally, Fig. 11 is a graph showing the results of measurement of the tumor blood volume in the trigeminal schwannoma on each examination day based on the results of contrast-enhanced head CT examination. In Fig. 11, the arrow indicates the time at which the administration of the vaccine composition began. As a result thereof, it was demonstrated that the tumor blood volume decreased after the peptide vaccine composition was administered.

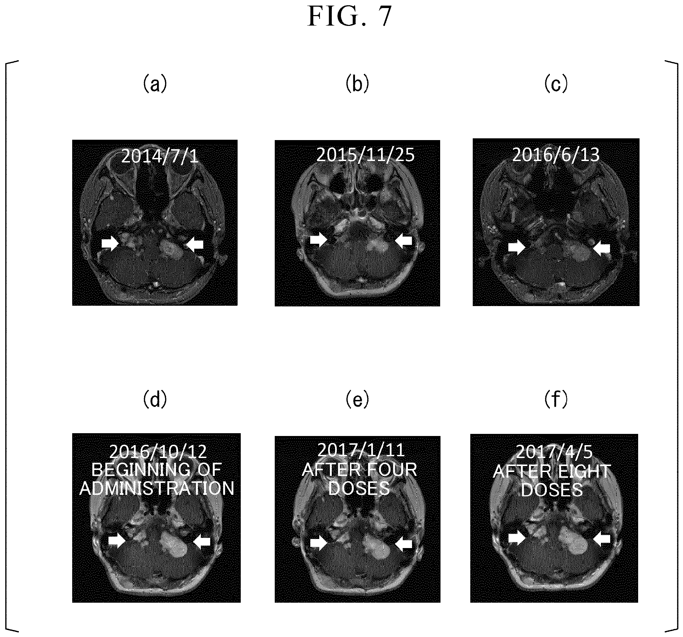

Additionally, Fig. 7(a) to (f) show photographs indicating the results of imaging of the right and left vestibular schwannomas in the patient in Case 2 by means of contrast-enhanced head MRI examination. The dates on which the images were taken are indicated on the upper part of each photograph. Fig. 7(a) to (c) show the results before administration of the peptide vaccine composition, Fig. 7(d) shows the results at the time the administration of the peptide vaccine composition began, Fig. 7(e) shows the results after four doses of the peptide vaccine composition were administered, and Fig. 7(f) shows the results after eight doses of the peptide vaccine composition were administered. In Fig. 7(a) to (f), the arrows indicate vestibular schwannomas.

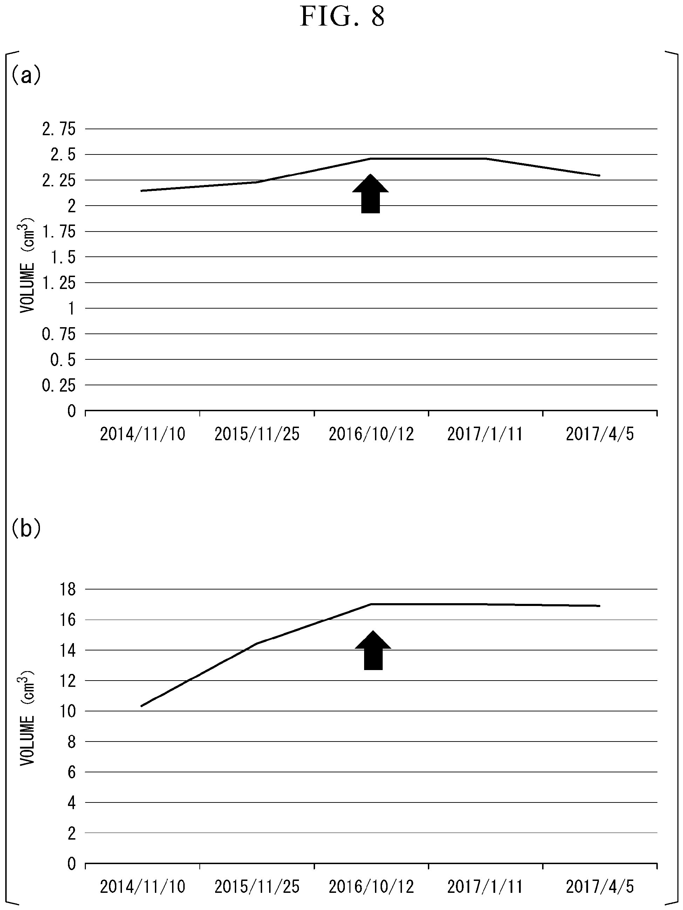

Additionally, Fig. 8(a) is a graph showing the results of measurement of the volume of the right vestibular schwannoma on each examination day, based on the results of the contrast-enhanced head MRI examination, and Fig. 8(b) is a graph showing the results of measurement of the volume of the left vestibular schwannoma on each examination day, based on the results of the contrast-enhanced head MRI examination. In Fig. 8(a) and (b), the arrows indicate the time at which the administration of the vaccine composition began.

As a result thereof, it was demonstrated that, after the administration of the peptide vaccine composition began, the volume of the right vestibular schwannoma, which had been on an increasing trend until that point, decreased by approximately 7% in comparison to that at the time the administration of the peptide vaccine composition began, and the growth of the left vestibular schwannoma stopped.

Additionally, Fig. 12(a) is a graph showing the results of measurement of the tumor blood volume in the right vestibular schwannoma on each examination day based on the results of contrast-enhanced head CT examination, and Fig. 12(b) is a graph showing the results of measurement of the tumor blood volume in the left vestibular schwannoma on each examination day based on the results of contrast-enhanced head CT examination. In Fig. 12(a) and (b), the arrows indicate the time at which the administration of the vaccine composition began. As a result thereof, it was demonstrated that the tumor blood volumes decreased after the peptide vaccine composition was administered.

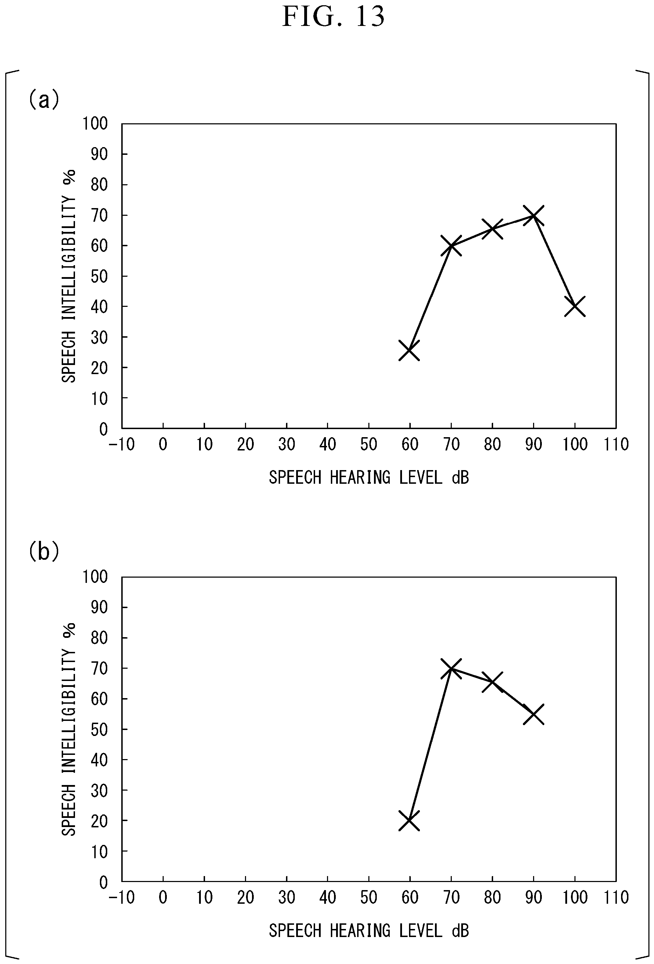

Additionally, Fig. 13(a) and (b) show graphs indicating the results of speech intelligibility tests performed on the patient in Case 2. A speech intelligibility test is a test in which a test subject is made to listen to speech, and it is measured how much of the language is intelligible, and how much the audio volume must be raised in order to make the speech intelligible. Fig. 13(a) shows the test results before administration of the peptide vaccine composition began, and Fig. 13(b) shows the test results after four doses of the peptide vaccine composition were administered.

The results of the speech intelligibility tests indicated a speech intelligibility of 60% at 70dB before the administration of the peptide vaccine composition began, whereas the intelligibility was approximately 70% at 70dB after four doses of the peptide vaccine composition were administered, so an improvement in hearing was observed.

Furthermore, as a result of analyzing reactions in cytotoxic T-cells (CTLs) specific to VEGFR-1 and VEGFR-2 in blood by means of the ELISPOT method, it was observed that CTLs against VEGFR-1 and VEGFR-2 were induced after the peptide vaccine composition was administered.

### [Experimental Example 4]

### (Administration of peptide vaccine composition to patient in Case 5)

The patient in Case 5 was a 41-year-old female. The HLA type of the patient in Case 5 was HLA-A^{∗}24:02. At the age of 23, this patient developed bilateral acoustic neuromas and was diagnosed with NF2. She underwent surgery on the right acoustic neuroma at the age of 23. She further underwent surgery at the age of 26. The hearing in the right ear declined after the operation, and the hearing in the left ear also went into a declining trend. In an MRI in March 2015, increasing trends were exhibited in the bilateral acoustic neuromas and in a meningioma in the rear surface of the left petrous bone. The administration of a peptide vaccine composition prepared in the same manner as in Experimental Example 1 was begun. The dosing schedule and the image analysis were the same as those in Experimental Example 2.

### (Image analysis)

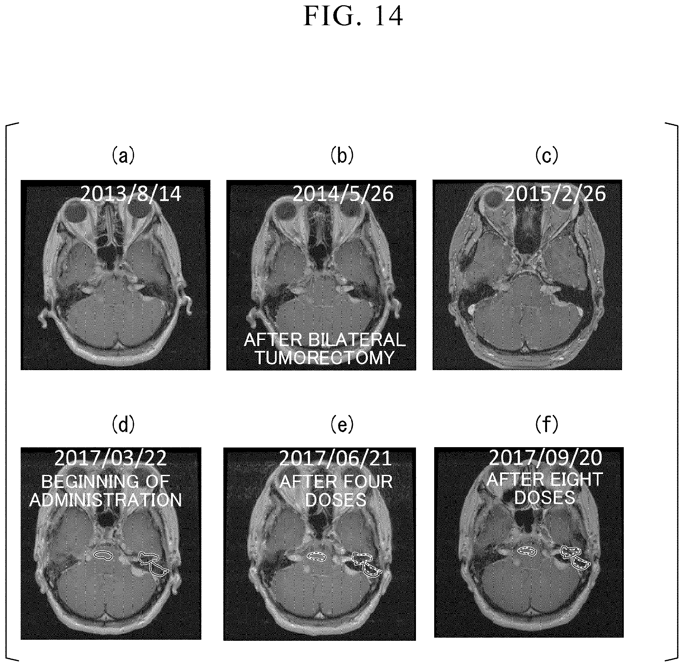

The effects of the administration of the peptide vaccine composition on the patient's tumors were investigated by means of contrast-enhanced head MRI examination. Fig. 14(a) to (f) show photographs indicating the results of imaging of the left vestibular schwannoma and the meningioma in the patient in Case 5 by means of contrast-enhanced head MRI examination. The dates on which the images were taken are indicated on the upper part of each photograph.

Fig. 14(a) to (d) show the results before administration of the peptide vaccine composition, Fig. 14(e) shows the results after four doses of the peptide vaccine composition were administered, and Fig. 14(f) shows the results after eight doses of the peptide vaccine composition were administered.

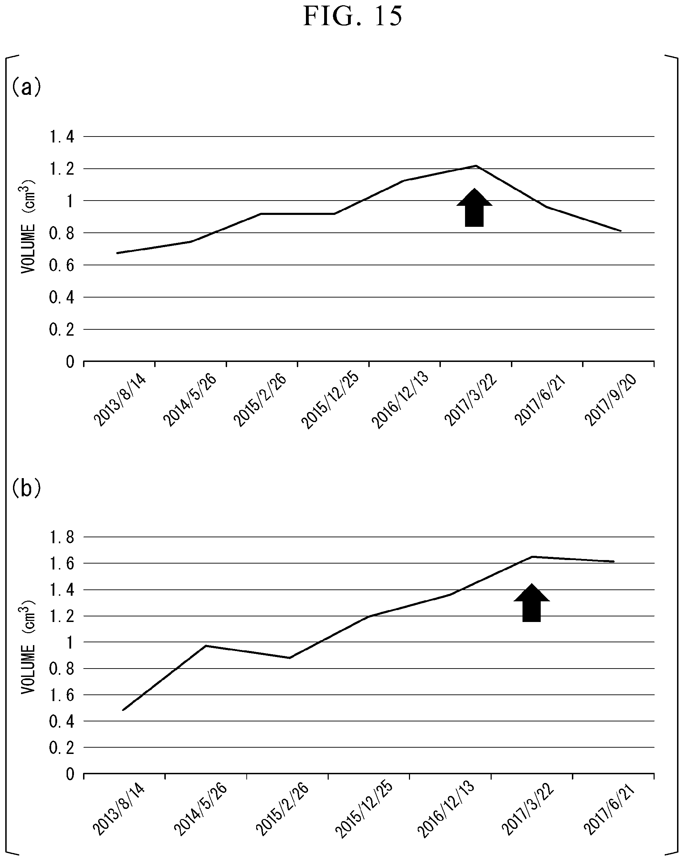

Additionally, Fig. 15(a) is a graph showing the results of measurement of the volume of the left vestibular schwannoma on each examination day, based on the results of the contrast-enhanced head MRI examination. Fig. 15(b) is a graph showing the results of measurement of the volume of the meningioma on each examination day, based on the results of the contrast-enhanced head MRI examination. In Fig. 15(a) and (b), the arrows indicate the time at which the administration of the vaccine composition began.

As a result thereof, after the peptide vaccine composition was administered, the volume of the left vestibular schwannoma, which had been on an increasing trend until that point, was reduced by 34%. Additionally, the volume of the meningioma, which had been on an increasing trend until that point, was reduced by 10%, and an increase-suppressing trend was exhibited thereafter.

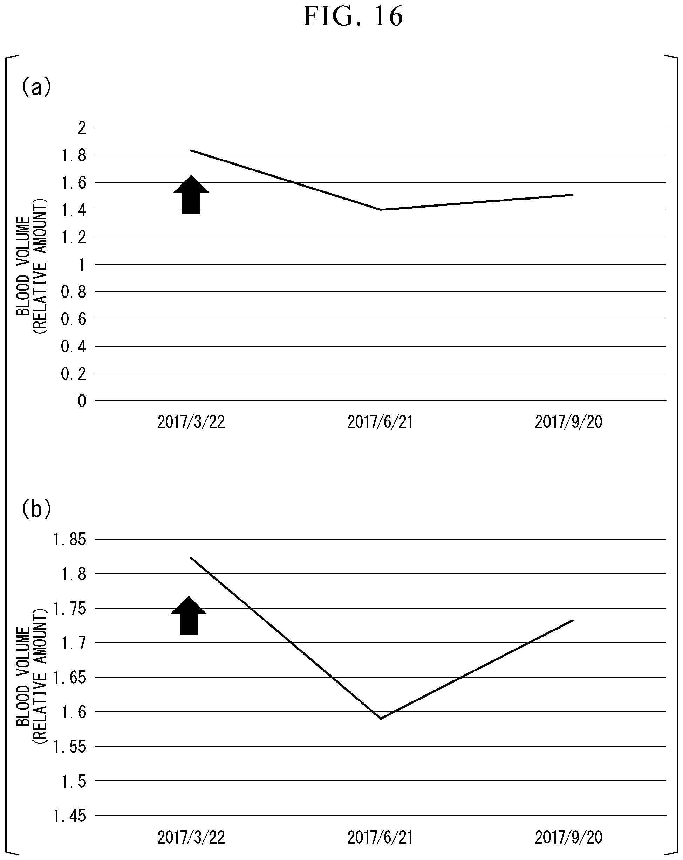

Additionally, Fig. 16(a) is a graph showing the results of measurement of the tumor blood volume in the left vestibular schwannoma on each examination day based on the results of contrast-enhanced head CT examination, and Fig. 16(b) is a graph showing the results of measurement of the tumor blood volume in the meningioma on each examination day based on the results of contrast-enhanced head CT examination. In Fig. 16(a) and (b), the arrows indicate the time at which the administration of the vaccine composition began. As a result thereof, it was demonstrated that the tumor blood volume exhibited a decreasing trend after the peptide vaccine composition was administered.

Additionally, pure-tone audiometry tests were performed on the patient in Case 5. A pure-tone audiometry test is a test in which the hearing with respect to seven different tones from 125 Hz to 8,000 Hz is measured to find the volume of the smallest audible tone. As a result thereof, an improvement trend was observed in the hearing in the left ear after eight doses of the peptide vaccine composition were administered.

Furthermore, as a result of analyzing reactions in cytotoxic T-cells (CTLs) specific to VEGFR-1 and VEGFR-2 in blood by means of the ELISPOT method, it was observed that CTLs against VEGFR-1 and VEGFR-2 were induced after the peptide vaccine composition was administered.

### [Experimental Example 5]

### (Preparation of peptide vaccine composition)

By mixing and emulsifying 2 mg of a peptide consisting of the amino acid sequence as set forth in SEQ ID NO:3, 2 mg of a peptide consisting of the amino acid sequence as set forth in SEQ ID NO:16 and 1 mL of incomplete Freund's adjuvant ("Montanide ISA51", SEPPIC), 2 mL of a peptide vaccine composition was prepared.

### [Experimental Example 6]

### (Administration of peptide vaccine composition to patient in Case 6)

The patient in Case 6 was a 23-year-old male. The HLA type of the patient in Case 6 was HLA-A^{∗}02:07. This patient had been experiencing tinnitus in the left ear since 2008. At the same time, a tumor developed on the skin and was removed, and NF2 was diagnosed. In 2011, bilateral vestibular schwannomas were discovered. Thereafter, the hearing in both ears gradually declined, and mild articulation difficulties began to occur. Additionally, a meningioma was observed in the anterior cranial base, and this thereafter exhibited a rapid increasing trend. The administration of a peptide vaccine composition prepared in the same manner as in Experimental Example 5 was begun. The dosing schedule and the image analysis were the same as those in Experimental Example 2.

### (Image analysis)

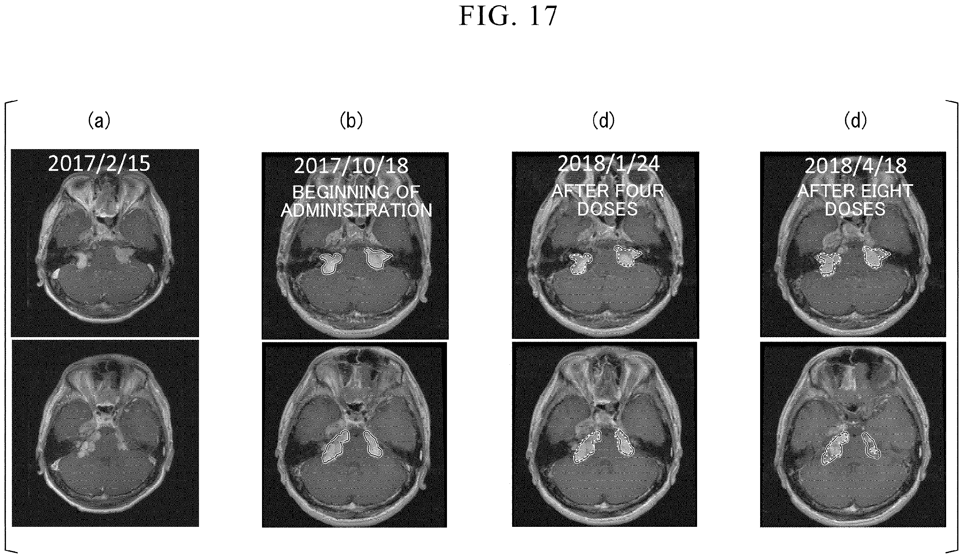

The effects of the administration of the peptide vaccine composition on the patient's tumors were investigated by means of contrast-enhanced head MRI examination. Fig. 17(a) to (d) show photographs indicating the results of imaging of the bilateral vestibular schwannomas in the patient in Case 6 by means of contrast-enhanced head MRI examination. The dates on which the images were taken are indicated on the upper part of each photograph.

Fig. 17(a) shows the results before administration of the peptide vaccine composition, Fig. 17(b) shows the results before administration of the peptide vaccine composition, Fig. 17(c) shows the results after four doses of the peptide vaccine composition were administered, and Fig. 17(d) shows the results after eight doses of the peptide vaccine composition were administered.

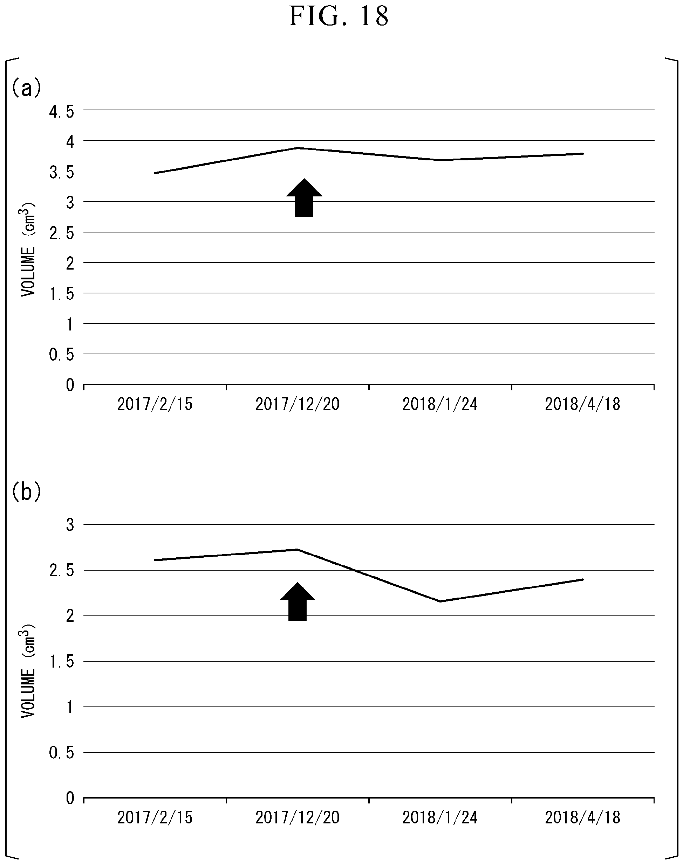

Additionally, Fig. 18(a) is a graph showing the results of measurement of the volume of the right vestibular schwannoma on each examination day, based on the results of the contrast-enhanced head MRI examination. Fig. 18(b) is a graph showing the results of measurement of the volume of the left vestibular schwannoma on each examination day, based on the results of the contrast-enhanced head MRI examination. In Fig. 18(a) and (b), the arrows indicate the time at which the administration of the vaccine composition began.

As a result thereof, after the peptide vaccine composition was administered, the volume of the right vestibular schwannoma, which had been on an increasing trend until that point, was reduced by 5%. Additionally, the volume of the left vestibular schwannoma, which had been on an increasing trend until that point, was reduced by 13%.

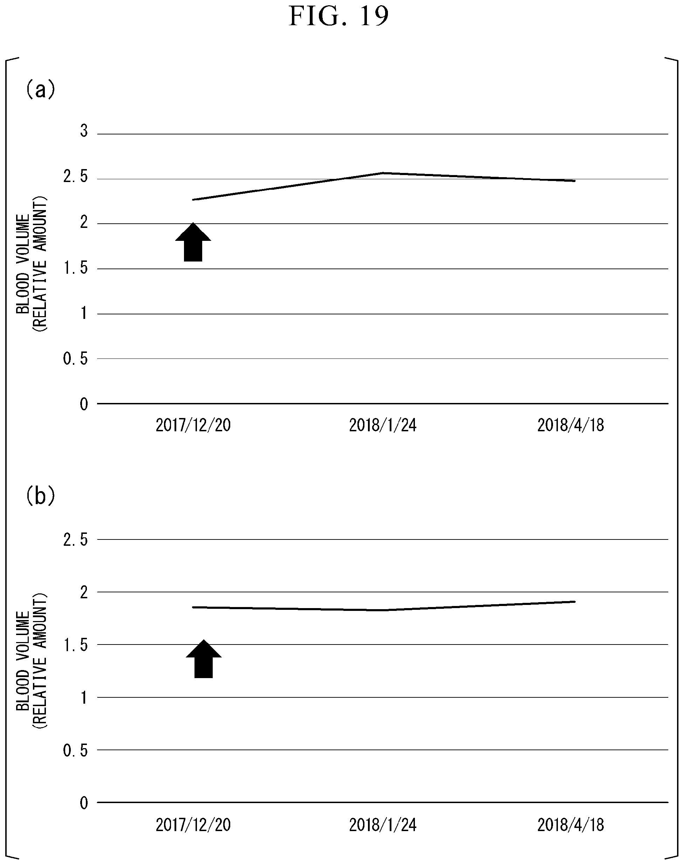

Additionally, Fig. 19(a) is a graph showing the results of measurement of the tumor blood volume in the right vestibular schwannoma on each examination day based on the results of contrast-enhanced head CT examination, and Fig. 19(b) is a graph showing the results of measurement of the tumor blood volume in the left vestibular schwannoma on each examination day based on the results of contrast-enhanced head CT examination. In Fig. 19(a) and (b), the arrows indicate the time at which the administration of the vaccine composition began. As a result thereof, it was demonstrated that the tumor blood volume in the right vestibular schwannoma exhibited a decreasing trend after the peptide vaccine composition was administered.

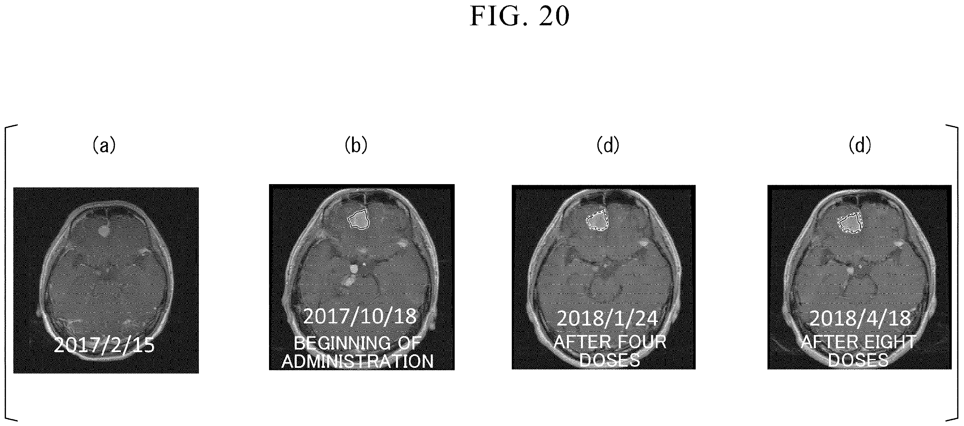

Additionally, Fig. 20(a) to (d) show photographs indicating the results of imaging of the meningioma in the patient in Case 6 by means of contrast-enhanced head MRI examination. The dates on which the images were taken are indicated on the upper part of each photograph.

Fig. 20(a) and (b) show the results before administration of the peptide vaccine composition, Fig. 20(c) shows the results after four doses of the peptide vaccine composition were administered, and Fig. 20(d) shows the results after eight doses of the peptide vaccine composition were administered.

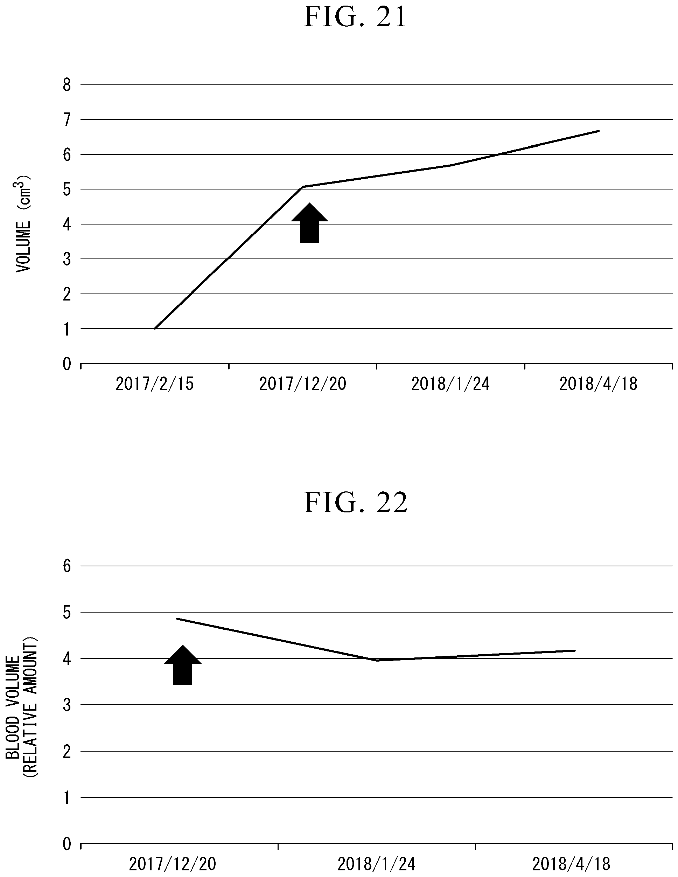

Additionally, Fig. 21 is a graph showing the results of measurement of the volume of the meningioma on each examination day based on the results of contrast-enhanced head MRI examination. In Fig. 21, the arrow indicates the time at which the administration of the vaccine composition began. As a result thereof, the increase rate in the meningioma tumor volume, which had been on a rapidly increasing trend until that point, was reduced.

Additionally, Fig. 22 is a graph showing the results of measurement of the tumor blood volume in the meningioma on each examination day based on the results of contrast-enhanced head CT examination. In Fig. 22, the arrow indicates the time at which the administration of the vaccine composition began. As a result thereof, it was demonstrated that the tumor blood volume in the meningioma exhibited a decreasing trend after the peptide vaccine composition was administered.

From the above results, the efficacy of the HLA-A^{∗}02:01-binding peptide vaccine against schwannomas and meningiomas in HLA-A^{∗}02:07 patients was demonstrated.

### [Experimental Example 7]

### (Histological analysis)

The expression levels of VEGF-A, VEGFR-1 and VEGFR-2 in tumor tissues before and after administration of the peptide vaccine composition to the patient in Case 1 were measured quantitatively by RT-PCR.

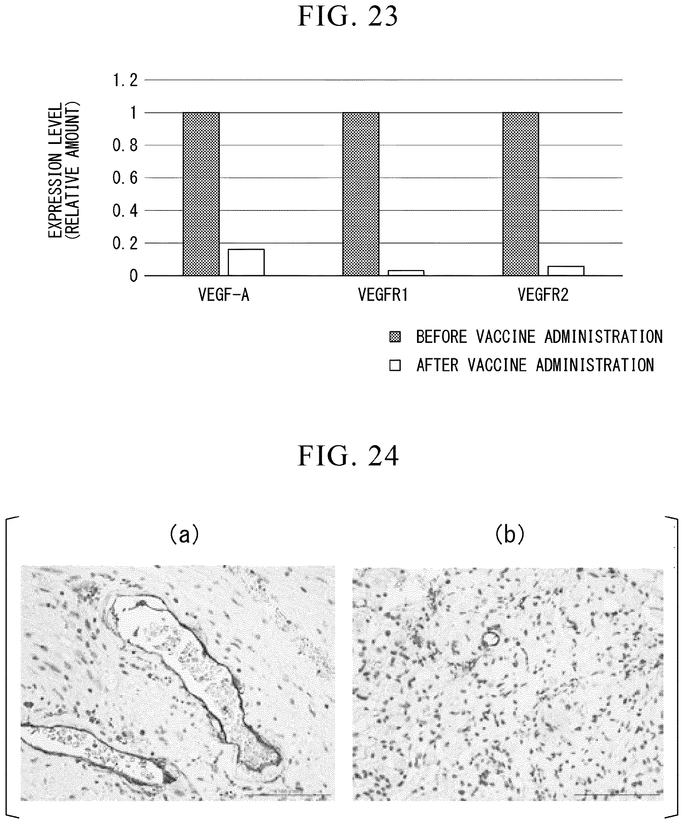

Fig. 23 shows a graph indicating the results of quantitative RT-PCR. As a result thereof, it was demonstrated that the expression levels of VEGF-A, VEGFR-1 and VEGFR-2 decreased after the peptide vaccine composition was administered.

Subsequently, tumor tissue sections taken before and after administration of the peptide vaccine composition to the patient in Case 1 were immunostained to analyze the expression of CD34. The expression of CD34 indicates the presence of vascular endothelial cells.

Fig. 24(a) and (b) show microscope photographs indicating the results of immunostaining. The magnification level was 40-times magnification. Fig. 24(a) shows the results obtained before administration of the peptide vaccine composition and Fig. 24(b) shows the results obtained after administration of the peptide vaccine composition. As a result thereof, it was demonstrated that, before the peptide vaccine composition was administered, many large blood vessels were observed, whereas, after the peptide vaccine was administered, large blood vessels were mostly unobserved.

Subsequently, tumor tissue sections taken before and after administration of the peptide vaccine composition to the patient in Case 1 were immunostained to detect the presence of FOXP3-positive cells. FOXP3-positive cells are regulatory T-cells.

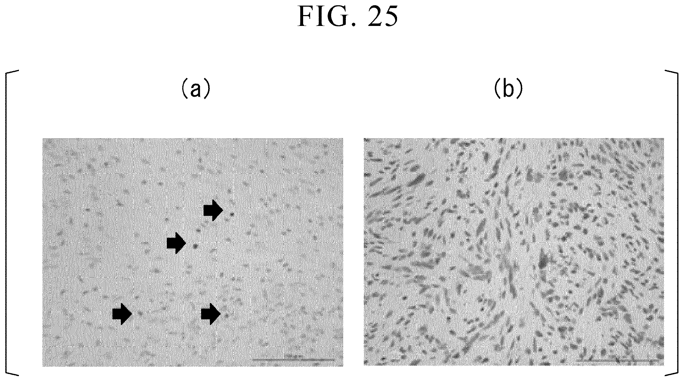

Fig. 25(a) and (b) show microscope photographs indicating the results of immunostaining. The magnification level was 40-times magnification. Fig. 25(a) shows the results obtained before administration of the peptide vaccine composition and Fig. 25(b) shows the results obtained after administration of the peptide vaccine composition. In Fig. 25(a) and (b), the arrows indicate the presence of FOXP3-positive cells. As a result thereof, it was demonstrated that, before the peptide vaccine composition was administered, the presence of regulatory T-cells was observed, whereas, after the peptide vaccine was administered, the presence of regulatory T cells was not observed.

Subsequently, tumor tissue sections taken before and after administration of the peptide vaccine composition to the patient in Case 1 were immunostained to triple-stain VEGFR-1, VEGFR-2 and PDGFR-β.

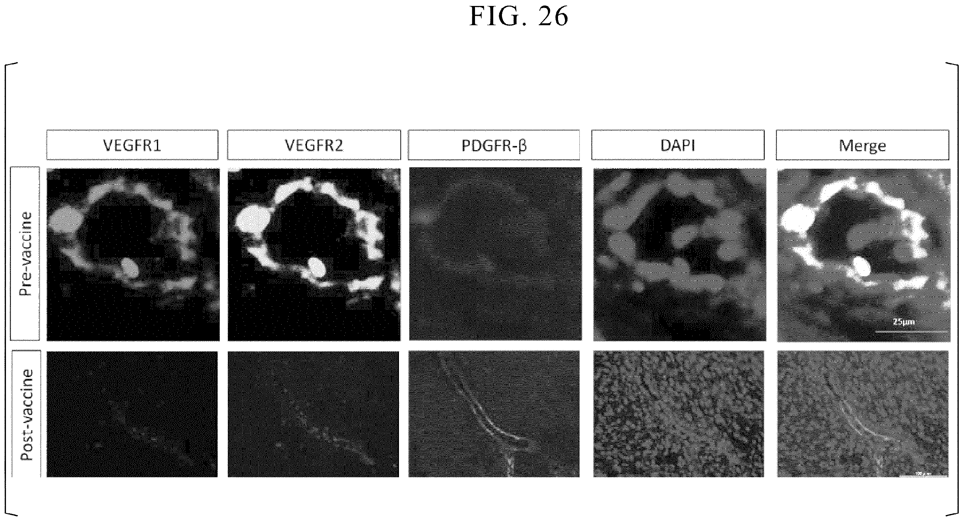

Fig. 26 shows a fluorescence microscope photograph indicating the results of immunostaining. In Fig. 26, "Pre-vaccine" indicates results obtained before the administration of the peptide vaccine composition, and "Post-vaccine" indicates results obtained after the administration of the peptide vaccine. Additionally, "DAPI" indicates results obtained by staining nuclei with 4',6-diamidino-2-phenylindole, and "Merge" indicates that the results have been obtained by merging the images.

As a result thereof, it was demonstrated that, before the peptide vaccine composition was administered, pericytes were missing, and many giant blood vessels in which VEGFR-1 and VEGFR-2 were highly expressed were formed, whereas, after the peptide vaccine composition was administered, there were many small blood vessels covered by pericytes.

### Industrial Applicability

According to the present invention, it is possible to provide a peptide vaccine and a peptide vaccine composition that are able to effectively treat cranial nerve disease.

## Claims

1. A peptide vaccine for cranial nerve disease, comprising a peptide that induces cytotoxic T-cells (CTLs) against Vascular Endothelial Growth Factor Receptor (VEGFR)-1-expressing cells, or a peptide that induces CTLs against VEGFR-2-expressing cells.

2. The peptide vaccine according to Claim 1, wherein the peptide that induces CTLs against VEGFR-1-expressing cells is an HLA-A^{∗}24:02-binding peptide.

3. The peptide vaccine according to Claim 2, wherein the peptide that induces CTLs against VEGFR-1-expressing cells consists of the amino acid sequence as set forth in SEQ ID NO:1, or an amino acid sequence obtained by deletion, substitution or addition of one or a plurality of amino acids with respect to the amino acid sequence as set forth in SEQ ID NO:1.

4. The peptide vaccine according to Claim 1, wherein the peptide that induces CTLs against VEGFR-1-expressing cells is an HLA-A^{∗}02:01-binding peptide.

5. The peptide vaccine according to Claim 4, wherein the peptide that induces CTLs against VEGFR-1-expressing cells consists of the amino acid sequence as set forth in any one of SEQ ID NO:2 to 4, or an amino acid sequence obtained by deletion, substitution or addition of one or a plurality of amino acids with respect to the amino acid sequence as set forth in any one of SEQ ID NO:2 to 4.

6. The peptide vaccine according to Claim 1, wherein the peptide that induces CTLs against VEGFR-1-expressing cells is an HLA-A^{∗}02:06-binding peptide or an HLA-A^{∗}02:07-binding peptide.

7. The peptide vaccine according to Claim 1, wherein the peptide that induces CTLs against VEGFR-2-expressing cells is an HLA-A^{∗}24:02-binding peptide.

8. The peptide vaccine according to Claim 7, wherein the peptide that induces CTLs against VEGFR-2-expressing cells consists of the amino acid sequence as set forth in any one of SEQ ID NO:5 to 10, or an amino acid sequence obtained by deletion, substitution or addition of one or a plurality of amino acids with respect to the amino acid sequence as set forth in any one of SEQ ID NO:5 to 10.

9. The peptide vaccine according to Claim 1, wherein the peptide that induces CTLs against VEGFR-2-expressing cells is an HLA-A^{∗}02:01-binding peptide.

10. The peptide vaccine according to Claim 9, wherein the peptide that induces CTLs against VEGFR-2-expressing cells consists of the amino acid sequence as set forth in any one of SEQ ID NO:11 to 16, or an amino acid sequence obtained by deletion, substitution or addition of one or a plurality of amino acids with respect to the amino acid sequence as set forth in any one of SEQ ID NO:11 to 16.

11. The peptide vaccine according to Claim 1, wherein the peptide that induces CTLs against VEGFR-2-expressing cells is an HLA-A^{∗}02:06-binding peptide or an HLA-A^{∗}02:07-binding peptide.

12. The peptide vaccine according to any one of Claims 1 to 11, wherein the cranial nerve disease is neurofibromatosis type II.

13. A peptide vaccine composition for cranial nerve disease, comprising the peptide vaccine according to any one of Claims 1 to 12, and a pharmaceutically acceptable carrier.


SEQUENCE LISTING

<110>  KEIO UNIVERSITY

<120>  PEPTIDE VACCINE AND PEPTIDE VACCINE COMPOSITION AGAINST CRANIAL
       NERVE DISEASE

<130>  PC-25730

<150>  JP2017-100361
<151>  2017-05-19

<160>  16

<170>  PatentIn version 3.5

<210>  1
<211>  9
<212>  PRT
<213>  Homo sapiens

<400>  1

Ser Tyr Gly Val Leu Leu Trp Glu Ile
1               5

<210>  2
<211>  9
<212>  PRT
<213>  Homo sapiens

<400>  2

Val Leu Leu Trp Glu Ile Phe Ser Leu
1               5

<210>  3
<211>  9
<212>  PRT
<213>  Homo sapiens

<400>  3

Thr Leu Phe Trp Leu Leu Leu Thr Leu
1               5

<210>  4
<211>  10
<212>  PRT
<213>  Homo sapiens

<400>  4

Asn Leu Thr Ala Thr Leu Ile Val Asn Val
1               5                   10

<210>  5
<211>  9
<212>  PRT

<213>  Homo sapiens

<400>  5

Arg Phe Val Pro Asp Gly Asn Arg Ile
1               5


<210>  6
<211>  9
<212>  PRT
<213>  Homo sapiens

<400>  6

Val Tyr Ser Ser Glu Glu Ala Glu Leu
1               5


<210>  7
<211>  9
<212>  PRT
<213>  Homo sapiens

<400>  7

Gly Tyr Arg Ile Tyr Asp Val Val Leu
1               5


<210>  8
<211>  9
<212>  PRT
<213>  Homo sapiens

<400>  8

Ser Tyr Met Ile Ser Tyr Ala Gly Met
1               5


<210>  9
<211>  9
<212>  PRT
<213>  Homo sapiens

<400>  9

Lys Trp Glu Phe Pro Arg Asp Arg Leu
1               5


<210>  10
<211>  9
<212>  PRT
<213>  Homo sapiens

<400>  10

Asp Phe Leu Thr Leu Glu His Leu Ile
1               5

<210>  11
<211>  9
<212>  PRT
<213>  Homo sapiens

<400>  11

Ala Met Phe Phe Trp Leu Leu Leu Val
1               5

<210>  12
<211>  9
<212>  PRT
<213>  Homo sapiens

<400>  12

Val Ile Ala Met Phe Phe Trp Leu Leu
1               5

<210>  13
<211>  9
<212>  PRT
<213>  Homo sapiens

<400>  13

Ala Val Ile Ala Met Phe Phe Trp Leu
1               5

<210>  14
<211>  9
<212>  PRT
<213>  Homo sapiens

<400>  14

Lys Leu Ile Glu Ile Gly Val Gln Thr
1               5

<210>  15
<211>  9
<212>  PRT
<213>  Homo sapiens

<400>  15

Tyr Met Ile Ser Tyr Ala Gly Met Val
1               5

<210>  16
<211>  9
<212>  PRT
<213>  Artificial Sequence

<220>
<223>  modified peptide

<400>  16

Val Leu Ala Met Phe Phe Trp Leu Leu
1               5